(19) Europäisches Patentamt
European Patent Office
Office européen des brevets

(11) Publication number: 0 365 210
A1

# (12) EUROPEAN PATENT APPLICATION

(21) Application number: 89310428.1

(22) Date of filing: 11.10.89

(51) Int. Cl.[5]: C07D 207/404 , C07D 207/38 , C07D 209/48 , C07D 211/88 , C07D 401/12 , C07D 409/12 , C07D 333/24 , C07D 333/38 , A61K 31/40 , A61K 31/44 , A61K 31/38

(30) Priority: 11.10.88 JP 255471/88

(43) Date of publication of application:
25.04.90 Bulletin 90/17

(84) Designated Contracting States:
DE FR GB IT

(71) Applicant: KYOWA HAKKO KOGYO CO., LTD.
6-1, Ohtemachi Itchome
Chiyoda-ku Tokyo(JP)

(72) Inventor: Kasai, Masaji c/o Patent Department
Kyowa Hakko Kogyo Co. Ltd. 6-1, Ohtemachi Itchome
Chiyoda-ku Tokyo(JP)
Inventor: Saito, Hiromitsu c/o Patent Department
Kyowa Hakko Kogyo Co. Ltd. 6-1, Ohtemachi Itchome
Chiyoda-ku Tokyo(JP)
Inventor: Murakata, Chikara c/o Patent Department
Kyowa Hakko Kogyo Co. Ltd. 6-1, Ohtemachi Itchome
Chiyoda-ku Tokyo(JP)
Inventor: Kitamura, Shigeto c/o Patent Department
Kyowa Hakko Kogyo Co. Ltd. 6-1, Ohtemachi Itchome
Chiyoda-ku Tokyo(JP)
Inventor: Yamada, Koji c/o Patent Department
Kyowa Hakko Kogyo Co. Ltd. 6-1, Ohtemachi Itchome
Chiyoda-ku Tokyo(JP)

(74) Representative: Lambert, Hugh Richmond et al
D. YOUNG & CO. 10 Staple Inn
London, WC1V 7RD(GB)

## (54) Hydroxamic acid derivatives.

(57) Hydroxamic acid derivatives represented by the general formula:
$R^1\text{-X-}(CH_2)_\ell\text{-}R^2$
and the general formula:
$R^1\text{-CON}(OH)\text{-}(CH_2)_{11}H$
exhibit a potent lipoxygenase inhibitory activity and are useful for the treatment and prevention of bronchial asthma, various allergic symptoms (allergic rhinitis, urticaria, etc.), ischemic heart disorders, hypertension, ischemic brain disorders, arteriosclerosis, inflammatory diseases, etc.

EP 0 365 210 A1

Xerox Copy Centre

## HYDROXAMIC ACID DERIVATIVES

The present invention relates to novel hydroxamic acid derivatives having lipoxygenase inhibitory activity. The compounds obtained by the present invention can be used in the prevention and treatment of diseases caused by lipoxygenase-mediated metabolites.

Lipoxygenase (EC. 1. 13. 11. 12) is an enzyme which is present in platelets, leucocytes, lymphocytes, etc. and catalyzes conversion of a polyvalent unsaturated fatty acid (especially arachidonic acid) into hydroperoxy acid. As the sites for introducing the hydroperoxy group into arachidonic acid by lipoxygenase, the 5-, 8-, 9-, 11-, 12- and 15-positions are known. For example, lipoxygenase which is present abundantly in platelets is 12-lipoxygenase that hydroperoxidizes the 12-position of arachidonic acid. 5-Lipoxygenase and 15-lipoxygenase are reported to be present in leucocytes. Hydroperoxyeicosatetraenoic acid produced from arachidonic acid by the action of lipoxygenase is unstable and converted into hydroxyeicosatetraenoic acid.

It has been revealed that these fatty acids produced by the action of lipoxygenase and their metabolites in vivo all have various physiological activities. For example, the entity of the slow reacting substance of anaphylaxis which is produced in the lung of a guinea pig with anaphylaxis or in human lung upon asthmatic attack and strongly contracts the bronchial smooth muscle slowly has been revealed to be leukotrienes C, D, E and F metabolized and produced from arachidonic acid via 5-lipoxygenase [Samuelson et al., Proc. Natl. Acad. Sci., 77, 2014(1980)]. Further, 12-hydroperoxyeicosatetraenoic acid (12-HPETE) and 12-hydroxyeicosatetraenoic acid (12-HETE) which are metabolites of 12-lipoxygenase exhibit a variety of physiological activities such as leucocyte migration activity, neutrophile attracting activity, platelet thromboxane synthase inhibitory activity, prostacyclin synthase inhibitory activity, and smooth muscle cell migration activity [reference: Prostaglandins and Pathological Conditions, edited by Seiitsu Murota, published by TOKYO KAGAKU DOJIN Publishing Co. (1984)].

As described above, it is reported that the metabolites of lipoxygenase contract various smooth muscles, for example, the smooth muscles of respiratory system (trachea, bronchus, lung tissue), blood vessel system and digestion organs, accelerate permeability of peripheral blood vessels, and exhibit leucocyte migration activity to cause bronchial asthma, allergic diseases (atopic dermatitis, visceral inflammation, etc.) and circulatory system diseases (edema, ischemic heart diseases, hypertension, ischemic brain disorders, arteriosclerosis, etc.) and are also chemical transmitters causing inflammatory diseases. It is thus considered that if lipoxygenase activity can be artificially prevented by its specific inhibitor, the prevention and treatment of the diseases described above will become possible.

As the compounds showing lipoxygenase inhibitory activity, AA-861, BW-755C and the like are known. BW-755C possesses pharmacological activities such as antiinflammatory activity, and AA-861 possesses pharmacological activities such as bronchial contraction inhibitory activity, antiinflammatory activity and anti-myocardial infarction activity [T. Yoshimoto et al., Biochem. Biophys. Acta, 713, 470 (1982); G.A. Higgs et al., Biochem. Pharmacol., 28, 1959 (1979); Y. Maki et al., Prostaglandins, 26 (6), 955 (1983); K. Sasaki et al., Adv. Prostaglandin, Thromboxane, Leukotriene Res., 17, 381 (1987)].

Compounds which are hydroxamic acid derivatives and which have lipoxygenase inhibitory activity are disclosed in Japanese Published Unexamined Patent Application No. 229845/1986 (US Patent No. 4,604,407, European Publication No. 0 200 377) and Japanese Published Unexamined Patent Application No. 25142/1986 (US Patent No. 4,623,661, European Publication No. 0 199 151). Further, 5,11-diaza-11-hydroxy-4-oxo-undecanoic acid is described in Japanese Patent Application No. 311692/1987 (European Publication No. 0 320 000).

In accordance with the present invention, novel compounds have been discovered having lipoxygenase inhibitory activity and which are therefore potentially useful as anti-allergic agents, anti-inflamatory agents, etc. It has to be acknowledged, however, that certain of these compounds are disclosed in Japanese Published Unexamined Patent Application No. 229845/1986, but no illustrative embodiments are given. These are compounds represented by the general formula:

$$R^1\text{-CO}\underset{\text{OH}}{\text{N}}\text{-}\ (CH_2)_{11}H$$

wherein $R^1$ represents methyl, n-propyl or phenyl.

In general terms, the present invention relates to hydroxamic acid derivatives represented by general formula [I]:

$$R^1\text{-X-}(CH_2)_\ell\text{-}R^2 \quad \cdot [I]$$

wherein: when $R^2$ is

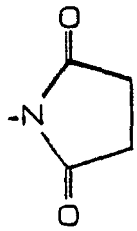, 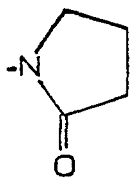, 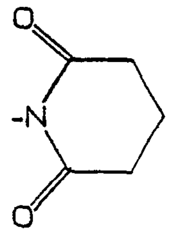, or

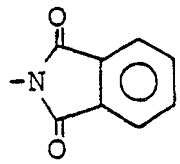,

$R^1$ represents a $C_1$-$C_{15}$ alkyl group,

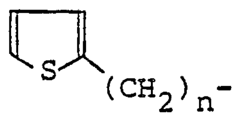

(wherein n is an integer of 0 to 4),

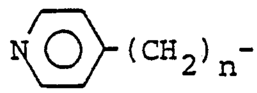

(wherein n is an integer of 0 to 4), phenyl or $R^3-\underset{\underset{O}{\|}}{C}-(CH_2)_m-$

(wherein m is an integer of 1 to 4, and $R^3$ represents hydroxy, $C_1$-$C_5$ alkoxy or phenylamino); and when $R^2$ is hydrogen, $R^1$ represents

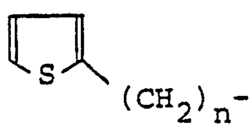

(wherein n is an integer of 0 to 4),

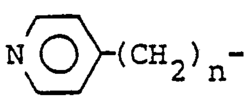

(wherein n is an integer of 0 to 4) or $R^3-\underset{\underset{O}{\|}}{C}-(CH_2)_m-$

(wherein m is an integer of 1 to 4, and $R^3$ represents hydroxy, $C_1$-$C_5$ alkoxy or phenylamino); X represents $-CO\underset{OH}{N}-$ or $-\underset{OH}{N}CO-$;

and $\ell$ represents an integer of 1 to 15, provided that when $R^1$ is $R^3-\underset{\underset{O}{\|}}{C}-(CH_2)_m-$, X is $-CO\underset{OH}{N}$

and salts thereof.

The present invention also relates to hydroxamic acid derivatives represented by the formula [IA]:

$R^1$-CON- $(CH_2)_{11}H$ [IA]
OH

(wherein $R^1$ represents methyl, n-propyl or phenyl) and salts thereof, for use for the purpose specified, i.e. in the manufacture of pharmaceutical preparations having lipoxygenase inhibitory activity.

Hereinafter the compounds represented by formula [I] are referred to as Compounds [I]. The same shall apply to compounds represented by formulae with other numbers. The hydroxamic acid derivatives and salts thereof according to the present invention strongly inhibit 5-lipoxygenase and/or 12-lipoxygenase.

In the definition of $R^1$ in formula [I], the $C_1$-$C_{15}$ alkyl group is a straight-chain or branched alkyl group having 1 to 15 carbon atoms such as methyl, ethyl, n-propyl, i-propyl, n-butyl, i-butyl, n-pentyl, n-hexyl, n-heptyl, n-octyl, n-nonyl, n-decyl, n-undecyl, 9-methyldecyl, n-dodecyl, 9-methylundecyl, 10-methylundecyl, n-tridecyl, 11-methyldodecyl, n-tetradecyl, 11-methyltridecyl, 12-methyltridecyl, n-pentadecyl and 13-methyltetradecyl.

In the definition of $R^3$ in formula [I], the $C_1$-$C_5$ alkoxy is a straight-chain or branched alkoxy group having 1 to 5 carbon atoms such as methoxy, ethoxy, n-propoxy, i-propoxy, n-butoxy and n-pentyloxy.

In cases where Compounds [I] are acidic compounds, their base addition salts may be formed and in cases where Compounds [I] are basic compounds, their acid addition salts may be formed. Examples of such base addition salts are ammonium salt, alkali metal salts such as lithium salt, sodium salt and potassium salt; alkaline earth metal salts such as calcium salt and magnesium salt; salts with organic bases such as triethylamine, morpholine, piperidine and dicyclohexylamine; and salts with basic amino acids such as arginine and lysine. Examples of the acid addition salts of Compounds [I] include hydrochloride, hydrobromide, sulfate, nitrate, formate, acetate, benzoate, maleate, fumarate, succinate, tartrate, citrate, oxalate, methanesulfonate, toluenesulfonate, aspartate and glutamate. Pharmaceutically acceptable nontoxic salts are preferred, but other salts are also useful in isolation and purifica tion of the products.

The processes for preparing Compounds [I] are described below. In the following description, [I-1], [I-2], etc. are intended to be included in [I]. $R^{1a}$, $R^{2a}$, etc. are intended to be included in $R^1$, $R^2$, etc., respectively. Further, [I-2]a and [I-2]b are intended to be included in [I-2], and [I-2]ab is intended to be included in [I-2]a.

Compounds [I-1] represented by formula [I] wherein $R^1$ represents $C_1$-$C_{15}$ alkyl, $R^2$ represents

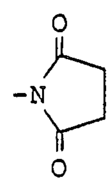 , 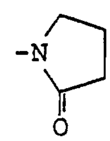 ,

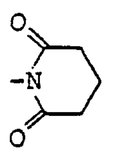 , or 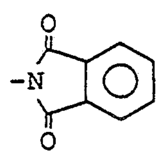 ,

and X represents -CON-
OH

can be prepared by the following Step 1.

[Step 1]:

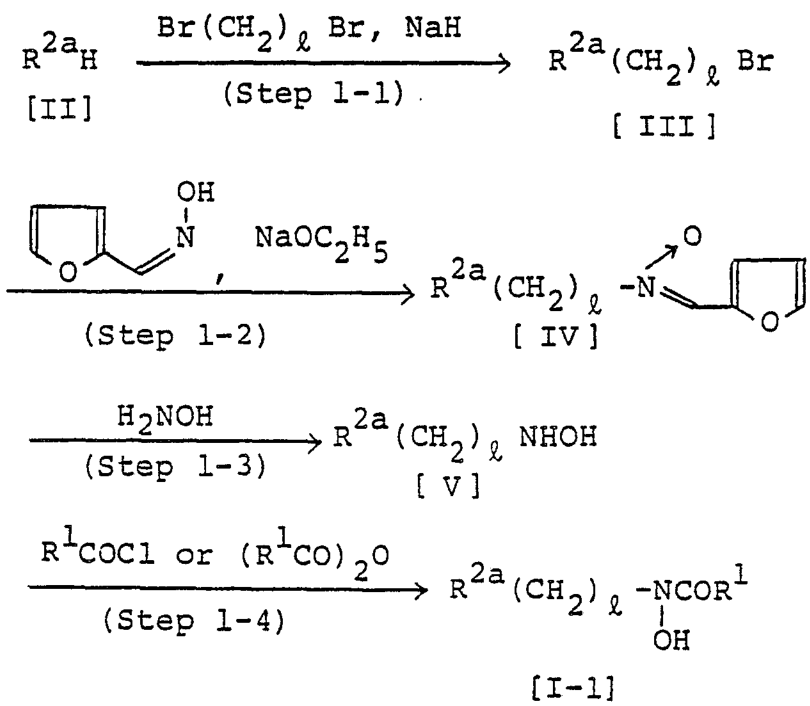


In the above formulae, $R^1$ represents $C_1$-$C_{15}$ alkyl; ℓ represents an integer of 1 to 15; and $R^{2a}$ represents

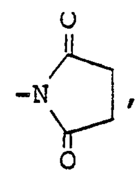,

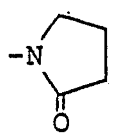, 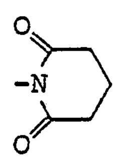 or 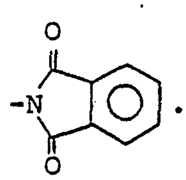.

Compound [III] is obtained by subjecting Compound [II] to reaction with a dibromoalkane in a solvent inert to the reaction, for example, dimethylformamide (DMF) in the presence of a base, for example, sodium hydride at 0 °C to room temperature for several hours. The dibromoalkane and the base are used in amounts of 1 to 3 equivalents and 1 to 1.5 equivalents, respectively, based on Compound [II] (Step 1-1).

Comound [IV] is obtained by subjecting Compound [III] to reaction with (Z)-2-furaldehyde oxime in a solvent inert to the reaction, for example, ethanol in the presence of a base, for example, sodium ethylate at 40 to 50 °C for several hours. (Z)-2-Furaldehyde oxime and sodium ethylate are respectively used in an amount of one equivalent based on Compound [III] (Step 1-2).

Compound [IV] is then allowed to react with hydroxylamine hydrochloride in a solvent inert to the reaction, for example, a solvent mixture of water and methanol at room temperature to 60 °C for 1 to 2 days to give Compound [V]. Hydroxylamine hydrochloride is used in an amount of 1 to 2 equivalents based on Compound [IV] (Step 1-3).

Compound [V] is allowed to react with an acid chloride or an acid anhydride in a solvent mixture of tetrahydrofuran (THF) and water at room temperature for several hours to give Compound [I-1]. The acid chloride or the acid anhydride is used in an amount of one equivalent based on Compound [V] (Step 1-4).

Compounds [I-2]a represented by formula [I] wherein $R^2$ is hydrogen, X represents $-CO\underset{OH}{N}-$ ,

and $R^{1a}$ reprepresents

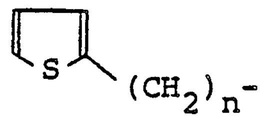

(wherein n is an integer of 0 to 4),

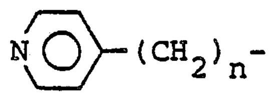

(wherein n is an integer of 0 to 4) or $R^3-\underset{\substack{\|\\O}}{C}-(CH_2)_n-$ (wherein n is an integer of 1 to 4 and $R^3$ is hydroxy or $C_1$-$C_5$ alkoxy) can be prepared by the following Step 2.

[Step 2]:

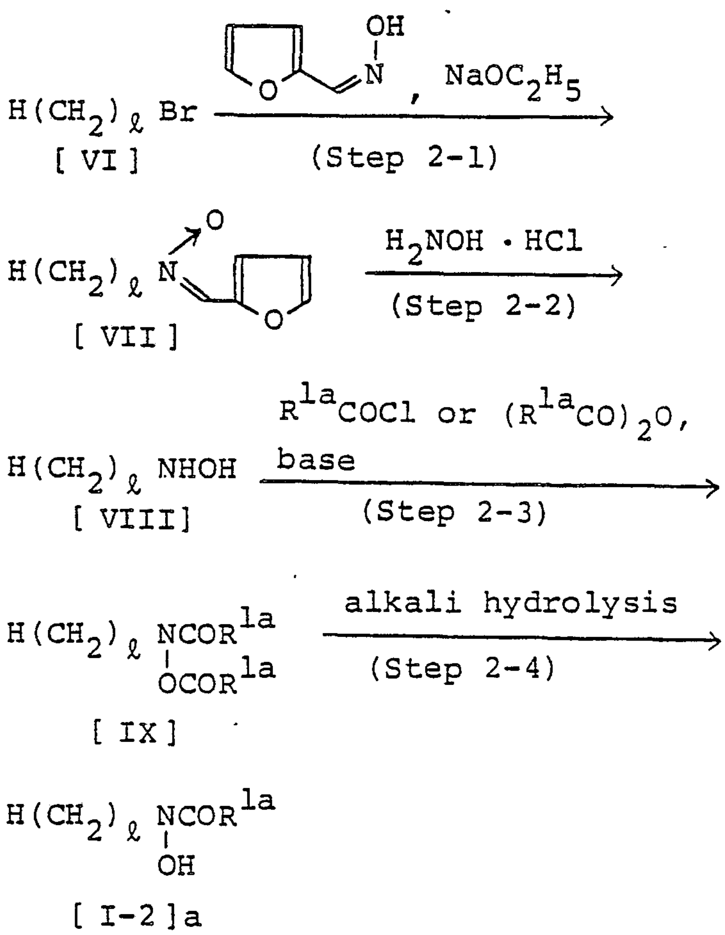


In the above formulae, $R^{1a}$ represents

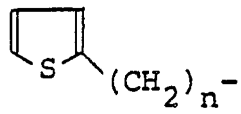

(wherein n is an integer of 0 to 4),

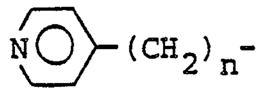 (wherein

n is an integer of 0 to 4) or $R^3-\underset{\underset{O}{\|}}{C}-(CH_2)_n-$ (wherein n is an integer of 1 to 4 and $R^3$ is hydroxy or $C_1$-$C_5$ alkoxy).

Compound [VII] is obtained by subjecting Compound [VI] to reaction with (Z)-2-furaldehyde oxime in a solvent inert to the reaction, for example, ethanol in the presence of a base, for example, sodium ethylate at 40 to 50 °C for several hours. (Z)-2-Furaldehyde oxime and sodium ethylate are respectively used in an amount of one equivalent based on Compound [VI] (Step 2-1).

Then, Compound [VII] is allowed to react with hydroxylamine hydrochloride in a solvent inert to the reaction, for example, a solvent mixture of water and methanol at room temperature to 60 °C for 1 to 2 days to give Compound [VIII]. Hydroxylamine hydrochloride is used in an amount of 1 to 2 equivalents based on Compound [VII] (Step 2-2).

Compound [VIII] is allowed to react with an acid chloride or an acid anhydride in a solvent inert to the reaction, for example, dichloromethane, THF or DMF in the presence of a base, for example, pyridine, triethylamine or sodium hydride at room temperature for several hours to give Compound [IX]. The acid chloride or the acid anhydride and the base are used in amounts of 2 to 5 equivalents and 1 to 5 equivalents, respectively, based on Compound [VIII] (Step 2-3).

Compound [IX] is then hydrolyzed in the presence of an alkali to give Compound [I-2]a. As the alkali, 28% aqueous ammonia, sodium hydroxide and the like are usually used. The alkali is used in large excess (more than 5 equivalents) based on Compound [IX]. The reaction is preferably carried out in the presence of methanol, THF, DMF, etc. at room temperature for one hour to one day (Step 2-4).

Compounds [I-2]b represented by formula [I] wherein $R^2$ is hydrogen, X is $-CO\underset{\underset{OH}{|}}{N}-$ , and $R^1$ represents

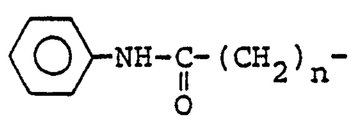

(wherein n is an integer of 1 to 4) can be prepared by the following Step 3.

[Step 3]:

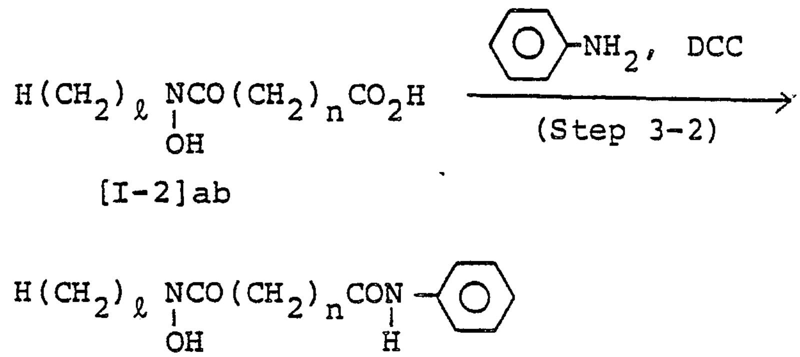


[I-2]ab

[I-2]b

In the above formulae, n is an integer of 1 to 4 and ℓ is an integer of 1 to 15.

Compound [I-2]ab is obtained in a similar manner as in Step 2 using $R^{1a}COCl$ or $(R^{1a}CO)_2O$ wherein $R^{1a}$ is $HO-\underset{\|}{\overset{}{C}}-(CH_2)_n-$ (with =O on C)

(n = 1 to 4) (Step 3-1).

Compound [I-2]ab is then allowed to react with phenylamine in a solvent inert to the reaction, for example, THF in the presence of dicyclohexylcarbodiimide (DCC) at 0 °C to room temperature overnight to give Compound [I-2]b. Phenylamine and DCC are used in amounts of 5 to 12 equivalents and 1 to 2 equivalents, respectively, based on Compound [I-2]ab (Step 3-2).

Compounds represented by the general formula:

$$R^1-CO\underset{OH}{N}-(CH_2)_{11}H$$

(wherein $R^1$ represents methyl, n-propyl or phenyl) can be obtained in a similar manner as in Step 2 except that compounds wherein $R^{1a}$ is methyl, n-propyl or phenyl are used.

After completion of the respective steps described above, the respective products can be isolated and purified by conventional methods used in organic synthesis, for example, by extraction, crystallization and chromatography in combination. In addition, the reaction products may also be used as starting materials for the subsequent step without purification.

The compounds of the present invention strongly inhibit lipoxygenase. Therefore, the compounds of the present invention are useful for the treatment and prevention of diseases caused by the metabolites of lipoxygenase, for example, bronchial asthma, various allergic symptoms (allergic rhinitis, urticaria, etc.), ischemic heart disorders, hypertension, ischemic brain disorders, arteriosclerosis, and inflammatory diseases. Dose is determined depending upon the intended therapeutic effect, route for administration, period for treatment, age and body weight of a patient, etc. The compounds may be administered orally or parenterally (injection, application, inhalation, etc.).

For administration, the compounds of the present invention may be used as they are. In general, however, the compounds are used in the form of tablets, pills, powders, granules, capsules, suppositories, injections, etc. Examples of the carriers used for preparing the pharmaceutical compositions include lactose, dextrose, sucrose, sorbitol, mannitol, glucose, cellulose, cyclodextrin, talc, starch, methyl cellulose, gelatin, gum arabic, polyethylene glycol, carboxymethyl cellulose, hydroxypropyl cellulose, sodium benzoate, sodium hydrogensulfite, aluminum stearate, magnesium stearate, mineral oil, vegetable oil, white vaseline and liquid paraffin. These carriers may be appropriately chosen depending upon the form of compositions.

The present invention is described below in more detail by referring to examples and an experimental example.

Table 1 shows the compounds obtained in the following examples.

Table 1

$$R^1 - X - (CH_2)_\ell - R^2$$

| Example No. | Compound No. | $R^1$ | X | $\ell$ | $R^2$ |
|---|---|---|---|---|---|
| 1 | 1 | $CH_3(CH_2)_{10}$ | -CON-<br>OH | 7 | 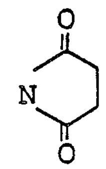 |
| 2 | 2 | $CH_3(CH_2)_{10}$ | -NCO-<br>OH | 5 | 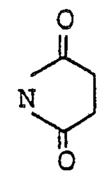 |
| 3 | 3 | $CH_3(CH_2)_{10}$ | -CON-<br>OH | 3 | 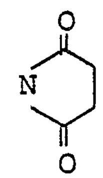 |
| 4 | 4 | $CH_3(CH_2)_{10}$ | -CON-<br>OH | 5 | 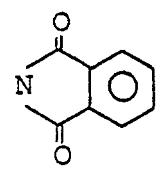 |

| Example No. | Compound No. | $R^1$ | X | ℓ | $R^2$ |
|---|---|---|---|---|---|
| 5 | 5 | $CH_3(CH_2)_{10}$ | -CON-<br>OH | 5 | 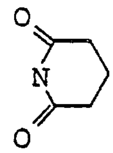 |
| 6 | 6 | $CH_3(CH_2)_{10}$ | -CON-<br>OH | 5 | 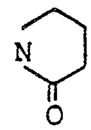 |
| 7 | 7 | 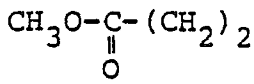 | -CON-<br>OH | 11 | H |
| 8 | 8 | $HOOC(CH_2)_2$ | -CON-<br>OH | 11 | H |
| 9 | 9 | 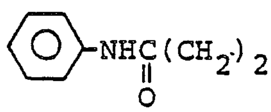 | -CON-<br>OH | 11 | H |
| 10 | 10 | $CH_3$ | -CON-<br>OH | 11 | H |
| 11 | 11 | $CH_3CH_2CH_2$ | -CON-<br>OH | 11 | H |
| 12 | 12 | 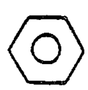 | -CON-<br>OH | 11 | H |
| 13 | 13 | 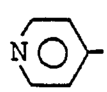 | -CON-<br>OH | 11 | H |
| 14 | 14 | 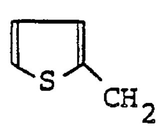 | -CON-<br>OH | 11 | H |

Example 1

In 30 ml of DMF were dissolved 2.97 g (0.03 mol) of succinimide and 1.32 g (0.033 mol) of 60% sodium hydride. Under ice cooling, 10.3 ml (0.06 mol) of 1,7-dibromoheptane was added to the solution, followed by stirring at room temperature for 3 hours. Ethyl acetate was added to the reaction mixture to effect extraction. The ethyl acetate layer was washed with water and then dried over anhydrous magnesium

sulfate, and the solvent was distilled away under reduced pressure. The residue was purified by silica gel column chromatography (eluting solvent: chloroform) to give 3.67 g (yield 45%) of Compound [III] wherein

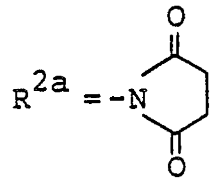

and $\ell = 7$ as a yellowish brown oily substance.

The physicochemical properties of the compound obtained above are as follows.

$^1$H-NMR (in $CDCl_3$) $\delta$ (ppm): 1.04-2.04 (m, 10H), 2.68(s, 4H), 3.38(t, 2H, J = 7Hz), 3.50(t, 2H, J = 8Hz)

Compound [III] wherein

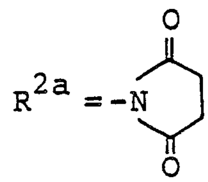

and $\ell = 7$ (3.59 g, 13.0 mmol) and 1.44 g (13.0 mmol) of (Z)-2-furaldehyde oxime were dissolved in 10 ml of ethanol. A solution of 299 mg of metallic sodium in 9.1 ml of ethanol was added to the solution, followed by stirring at 40° C for 4 hours. The solvent was distilled away under reduced pressure and chloroform was added to the residue. After washing with water, the mixture was dried over anhydrous magnesium sulfate. The solvent was distilled away under reduced pressure and the residue was purified by silica gel column chromatography (eluting solvent: 1% methanol/chloroform) to give 1.07 g (yield 27%) of Compound [IV] wherein

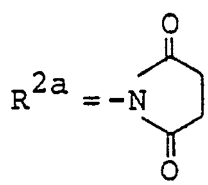

and $\ell = 7$.

The physicochemical properties of the compound obtained above are as follows.

$^1$H-NMR (in $CDCl_3$) $\delta$ (ppm): 1.20-2.20(m, 10H), 2.68(s, 4H), 3.48(t, 2H, J = 7Hz), 3.88(t, 2H, J = 7Hz), 6.52-6.64(m, 1H), 7.48(d, 1H, J = 2Hz), 7.57(s, 1H), 7.74(d, 1H, J = 4Hz)

Compound [IV] wherein

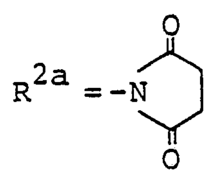

and $\ell = 7$ (970 mg, 3.17 mmol) and 220 mg (3.17 mmol) of hydroxylamine hydrochloride were suspended in a solvent mixture of 6 ml of methanol and 6 ml of water, followed by stirring at room temperature overnight. Water and ether were added to the reaction mixture to effect extraction. The water layer was adjusted to pH 8 with 1 N NaOH and then extracted with chloroform, and the chloroform layer was dried over anhydrous magnesium sulfate. After the solvent was distilled away under reduced pressure, recrystallization was carried out from a solvent mixture of ethyl acetate and n-hexane to give 330 mg (yield 46%) of Compound [V] wherein

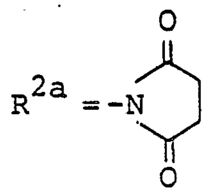

and $\ell = 7$ .

The physicochemical properties of the compound obtained above are as follows.

$^1$H-NMR (in $CDCl_3$) $\delta$ (ppm): 1.08-1.80 (m, 10H), 2.64(s, 4H), 2.92(t, 2H, J = 7Hz), 3.48(t, 2H, J = 7Hz)

Compound [V] wherein

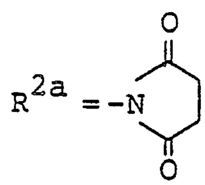

and $\ell = 7$ (229 mg, 1 mmol) and 219 mg (1 mmol) of n-dodecanoyl chloride were dissolved in a solvent mixture of 1 ml of THF and 0.2 ml of water, followed by stirring at room temperature for 2 hours. Chloroform was added to the reaction solution, and the resulting mixture was washed successively with 5% aqueous citric acid solution, saturated aqueous sodium hydrogencarbonate solution and saturated aqueous sodium chloride solution and then dried over anhydrous magnesium sulfate. After the solvent was distilled away under reduced pressure, the residue was recrystallized from a solvent mixture of ethyl acetate and n-hexane to give 320 mg (yield 78%) of Compound (1) as colorless needles having a melting point of 61.5 to 62 °C.

The physicochemical properties of Compound (1) are as follows.

$^1$H-NMR (in $CDCl_3$) $\delta$ (ppm): 0.88(br. t, 3H, J = 6Hz), 1.12-2.52(m, 30H), 2.72(s, 4H), 3.52(t, 2H, J = 7Hz), 3.62(t, 2H, J = 7Hz)

EIMS (m/z): 411 $(M+1)^+$, 410 $(M^+)$

## Example 2

In 45 ml of ethanol were dissolved 13.39 ml (60 mmol) of 1-bromoundecane and 6.66 g (60 mmol) of (Z)-2-furaldehyde oxime. A solution of 1.38 g of metallic sodium in 40 ml of ethanol was added to the solution, followed by stirring at 40 °C for 3 hours and 20 minutes. The reaction mixture was concentrated and the residue was dissolved in chloroform. The solution was washed with water three times and then dried over anhydrous magnesium sulfate. After the solvent was distilled away under reduced pressure, the residue was purified by silica gel column chromatography (eluting solvent: chloroform) to give 12.47 g (yield 79%) of Compound [VII] wherein $\ell = 11$.

The physicochemical properties of Compound [VII] wherein $\ell = 11$ are as follows.

$^1$H-NMR (in $CDCl_3$) $\delta$ (ppm): 0.88(m, 3H), 1.1-2.1(m, 18H), 3.88(t, 3H, J = 7Hz), 6.56(m, 1H), 7.48(m, 1H), 7.54(s, 1H), 7.76(d, 1H, J = 4Hz)

Compound [VII] wherein $\ell = 11$ was suspended in 100 ml of methanol and 70 ml of water, and 3.94 g of hydroxylamine hydrochloride was added to the suspension, followed by stirring at room temperature for 24 hours. Methanol was distilled away and an aqueous sodium hydrogencarbonate solution was added to the reaction mixture. The mixture was extracted with ethyl acetate and the extract was dried over anhydrous magnesium sulfate. After the solvent was distilled away, ether was added to the residue. Insoluble matters resulting from trituration were separated by filtration and dried to give 4.21 g (yield 48%) of Compound [VIII] wherein $\ell = 11$.

The physicochemical properties of Compound [VIII] wherein $\ell = 11$ are as follows.

$^1$H-NMR (in $CDCl_3$) $\delta$ (ppm): 0.89(br. t, 3H, J = 6Hz), 1.08-1.80(m, 18H), 2.96(t, 2H, J-7Hz), 5.00(br. s, 2H)

Compound [VIII] wherein $\ell = 11$ (188 mg, 1 mmol) and 1.22 g (3 mmol) of 6-succinimidocaproic anhydride were dissolved in dichloromethane, and 0.32 ml of pyridine was added to the solution. The mixture was stirred at room temperature for 2 days. The solvent was distilled away under reduced pressure,

and 5 ml of methanol and 0.44 ml of 28% aqueous ammonia were added to the residue. The mixture was stirred at room temperature for 3 hours. After the solvent was distilled away under reduced pressure, the residue was purified by silica gel column chromatography (eluting solvent: 1% methanol/chloroform) to give 217 mg (yield 57%) of Compound (2) as colorless needles having a melting point of 73.5 to 75 °C.

The physicochemical properties of Compound (2) are as follows.

$^1$H-NMR (in $CDCl_3$) $\delta$ (ppm): 0.88(t, 3H, J = 6Hz), 1.04-2.56(m, 26H), 2.70(s, 4H), 3.40-3.72(m, 4H)

EIMS (m/z): 383 $(M+1)^+$, 382 $(M^+)$

## Example 3

Compound [V] wherein

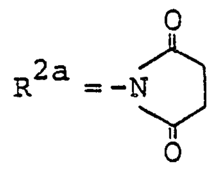

and $\ell = 3$ (200 mg, yield 2.4%) was obtained from succinimide in a similar manner as in Example 1 except that 1,3-dibromopropane was used in place of 1,7-dibromoheptane.

The physicochemical properties of the obtained compound are as follows.

$^1$H-NMR (in $CDCl_3$) $\delta$ (ppm): 1.84(m, 2H), 2.71(s, 4H), 2.92(t, 2H, J = 7Hz), 3.61(t, 2H, J = 7Hz), 5.06(br. s, 1H)

Compound (3) (37 mg, yield 35%) was obtained from Compound [V] wherein

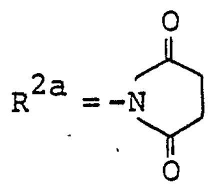

and $\ell = 3$ and n-dodecanoyl chloride as colorless needles having a melting point of 64.5 to 67 °C in a similar manner as in Example 1.

The physicochemical properties of Compound (3) are as follows.

$^1$H-NMR (in $CDCl_3$) $\delta$ (ppm): 0.88(br. t, 3H, J = 6Hz), 1.04-2.56(m, 22H), 2.72(s, 4H), 3.59(t, 2H, J = 7Hz), 3.65(t, 2H, J = 7Hz)

SIMS (m/z): 355 $(M+1)^+$

## Example 4

The hydrochloride of Compound [V] wherein

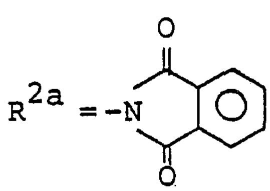

and $\ell = 5$ (15.52 g, yield 45%) was obtained in a similar manner as in Example 1 except that phthalimide and 1,5-dibromopentane were used in place of succinimide and 1,7-dibromoheptane, respectively.

The physicochemical properties of the obtained compound are as follows.

$^1$H-NMR (in $CD_3OD$) $\delta$ (ppm): 1.20-2.00(m, 6H), 3.23(t, 2H, J = 8Hz), 3.69(t, 2H, J = 7Hz), 7.83(s, 4H)

Compound (4) (1.07 g, yield 83%) was obtained from the hydrochloride of Compound [V] wherein

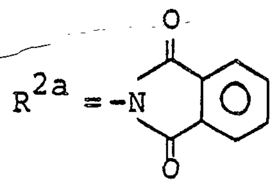

and $\ell = 5$ and n-dodecanoyl chloride as colorless needles having a melting point of 84 to 86 °C in a similar manner as in Example 1.

The physicochemical properties of Compound (4) are as follows.
$^1$H-NMR (in $CDCl_3$) $\delta$ (ppm): 0.80-2.52(m, 29H), 3.52-3.88(m, 4H), 7.64-8.00(m, 4H)
SIMS (m/z): 431 $(M+1)^+$

## Example 5

Compound [V] wherein

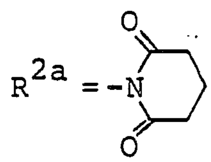

and $\ell = 5$ (1.07 g, yield 20%) was obtained in a similar manner as in Example 1 except that glutarimide and 1,5-dibromopentane were used in place of succinimide and 1,7-dibromoheptane, respectively.

The physicochemical properties of the obtained compound are as follows.
$^1$H-NMR (in $CDCl_3$) $\delta$ (ppm): 1.16-2.52(m, 12H), 2.94(br. t, 2H, J = 7Hz), 3.12-3.58(m, 2H)

Compound (5) (354 mg, yield 30%) was obtained from Compound [V] wherein

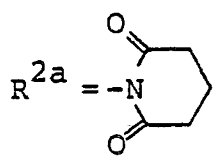

and $\ell = 5$ and n-dodecanoyl chloride as colorless needles having a melting point of 90 to 91 °C in a similar manner as in Example 1.

The physicochemical properties of Compound (5) are as follows.
$^1$H-NMR (in $CDCl_3$) $\delta$ (ppm): 0.87(br. t, 3H, J = 6Hz), 1.08-2.68(m, 32H), 3.12-3.40(m, 2H), 3.52-3.76 (m, 2H)
EIMS (m/z): 397 $(M^+)$

## Example 6

Compound [V] wherein

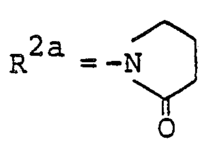

and $\ell = 5$ (3.79 g, yield 42%) was obtained in a similar manner as in Example 1 except that 2-pyrrolidinone and 1,5-dibromopentane were used in place of succinimide and 1,7-dibromoheptane, respectively.

The physicochemical properties of the obtained compound are as follows.
$^1$H-NMR (in $CDCl_3$) $\delta$ (ppm): 1.04-2.25(m, 8H), 2.39(t, 2H, J = 8Hz), 2.93(t, 2H, J = 7Hz), 3.29(t, 2H, J = 8Hz),

3.36(t, 2H, J=7Hz), 5.51(br. s, 2H)

Compound (6) (344 mg, yield 34%) was obtained from Compound [V] wherein

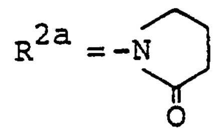

and $\ell$=5 and n-dodecanoyl chloride as colorless needles having a melting point of 49.5 to 51.5°C in a similar manner as in Example 1.

The physicochemical properties of Compound (6) are as follows.

$^1$H-NMR (in $CDCl_3$) $\delta$ (ppm): 0.68-2.72(m, 33H), 3.20-3.48(m, 4H), 3.63(m, 2H), 8.50(br. s, 1H)

EIMS (m/z): 368 ($M^+$)

Example 7

Compound (7) (24 mg, yield 100%) was obtained from Compound [VIII] wherein $\ell$=11 in a similar manner as in Example 2 except that carbomethoxypropionyl chloride was used in place of 6-succinimidocaproic anhydride.

The physicochemical properties of Compound (7) are as follows.

$^1$H-NMR (in $CDCl_3$) $\delta$ (ppm): 0.60-2.10(m, 21H), 2.44-2.88(m, 4H), 3.40-3.84(m, 2H), 3.71(s, 3H)

SIMS (m/z): 302 $(M+1)^+$

Example 8

Compound [VIII] wherein $\ell$=11 (187 mg, 1 mmol) and 80 mg (2 mmol) of 60% sodium hydride were dissolved in 5 ml of DMF, and 0.5 g (5 mmol) of succinic anhydride was added to the solution. The mixture was stirred at room temperature for 4 hours. Subsequently, the reaction mixture was treated in a similar manner as in Example 2 to give 176 mg (yield 61%) of Compound (8) as colorless needles having a melting point of 110 to 112°C.

The physicochemical properties of Compound (8) are as follows.

$^1$H-NMR (in $CDCl_3$) $\delta$ (ppm): 0.89(br. t, 3H, J=6Hz), 1.08-1.92(m, 18H), 2.60-2.92(m, 4H), 3.64(t, 2H, J=7Hz)

SIMS (m/z): 288 $(M+1)^+$

Example 9

Compound (8) (70 mg. 0.24 mmol) and 0.22 ml of aniline were dissolved in 1 ml of THF. Under ice cooling, 1 ml of THF solution containing 74 mg of DCC was added to the solution. The mixture was stirred at 0°C to room temperature overnight. The precipitate was filtered off and ethyl acetate was added to the filtrate. The mixture was washed successively with saturated aqueous sodium hydrogencarbonate solution, 5% aqueous citric acid solution and saturated aqueous sodium chloride solution, and then dried over anhydrous magnesium sulfate. After the solvent was distilled away under reduced pressure, the residue was purified by silica gel column chromatography (eluting solvent: 5% methanol/chloroform) to give 76 mg (yield 88%) of Compound (9) as colorless needles having a melting point of 136 to 137°C.

The physicochemical properties of Compound (9) are as follows.

$^1$H-NMR (in $CDCl_3$) $\delta$ (ppm): 0.72-2.20(m, 21H), 2.68-2.92(m, 4H), 2.64(t, 2H, J=7Hz), 7.00-7.64(m, 5H)

EIMS (m/z): 362 ($M^+$)

Example 10

Compound (10) (203 mg, yield 94%) was obtained from Compound [VIII] wherein $\ell$=11 as colorless needles having a melting point of 45 to 49°C in a similar manner as in Example 2 except that acetic

anhydride was used in place of 6-succinimidocaproic anhydride.

The physicochemical properties of Compound (10) are as follows.

$^1$H-NMR (in $CDCl_3$) δ (ppm): 0.88(br. t, 3H, J=6Hz), 1.08-2.00(m, 18H), 2.12(s, 3H), 3.62(t, 2H, J=7Hz)
EIMS (m/z): 229 ($M^+$)

Example 11

Compound (11) (218 mg, yield 85%) was obtained from Compound [VIII] wherein ℓ=11 as colorless needles having a melting point of 44 to 45°C in a similar manner as in Example 2 except that butyric anhydride was used in place of 6-succinimidocaproic anhydride.

The physicochemical properties of Compound (11) are as follows.

$^1$H-NMR (in $CDCl_3$) δ (ppm): 0.76-1.04(m, 3H), 0.99(t, 3H, J=7Hz), 1.12-2.04(m, 20H), 2.34(t, 2H, J=7Hz), 3.63(t, 2H, J=7Hz)
EIMS (m/z): 257 ($M^+$)

Example 12

Compound (12) (218 mg, yield 75%) was obtained from Compound [VIII] wherein ℓ=11 as colorless needles having a melting point of 69.5 to 71.5°C in a similar manner as in Example 2 except that benzoyl chloride was used in place of 6-succinimidocaproic anhydride.

The physicochemical properties of Compound (12) are as follows.

$^1$H-NMR (in $CDCl_3$) δ (ppm): 0.88(br. t, 3H, J=6Hz), 1.04-2.00(m, 18H), 3.65(t, 2H, J=8Hz), 7.48(s, 5H)
EIMS (m/z): 291 ($M^+$)

Example 13

Compound (13) (112 mg, yield 78%) was obtained from Compound [VIII] wherein ℓ=11 as colorless needles having a melting point of 97 to 97.5°C in a similar manner as in Example 2 except that isonicotinoyl chloride was used in place of 6-succinimidocaproic anhydride.

The physicochemical properties of Compound (13) are as follows.

$^1$H-NMR (in $CDCl_3$) δ (ppm): 0.88(br. t, 3H, J=6Hz), 1.08-2.00(m, 18H), 3.62(t, 2H, J=7Hz), 7.32-7.48 (m, 2H), 8.64-8.80(m, 2H)
EIMS (m/z): 292 ($M^+$)

Example 14

Compound (14) (79 mg, yield 51%) was obtained from Compound [VIII] wherein ℓ=11 in a similar manner as in Example 2 except that 2-thiopheneacetyl chloride was used in place of 6-succinimidocaproic anhydride.

The physicochemical properties of Compound (14) are as follows.

$^1$H-NMR (in $CDCl_3$) δ (ppm): 0.88(br. t, 3H, J=6Hz), 1.06-2.00(m, 18H), 3.54-4.10(m, 4H), 6.90-7.10 (m, 2H), 7.18-7.34(m, 1H)
EIMS (m/z): 311 ($M^+$)

Experimental Example

Lipoxygenase inhibitory activity of the compounds shown in Table 2 was determined by the following *in vitro* tests.

a) Method for determining the inhibitory activity against leucocyte 5-lipoxygenase:

Lipoxygenase activity was determined by a modification of the method of B.A. Jakschik *et al.* [Biochem.

Biophys. Res. Commun., 95, 103 (1980)]. That is, rat basophilic leukemia cells (RBL-1, ATCC No. CRL 1378) were used as the source of 5-lipoxygenase. The cells were contacted with a test compound in 60 µl of 0.17 M Trishydrochloride buffer (pH 7.4) containing 1.7 M calcium chloride, 3.3 mM adenosine triphosphate and 1 mM glutathione at 37° C for 5 minutes. Then, 50 µl of 60 µM arachidonic acid was added to the reaction mixture and the mixture was allowed to stand at 37° C for 5 minutes. To the mixture were added 200 µl of methanol and 13-hydroxylinoleic acid as the internal standard, followed by thorough shaking. The mixture was allowed to stand at -20° C for 30 minutes and then centrifuged at 12000 r.p.m. for 10 minutes. After the supernatant was evaporated to dryness in a stream of nitrogen, 200 µl of 75% aqueous methanol was added to the residue. The resulting solution (100 µl) was subjected to high performance liquid chromatography to quantitatively determine 5-HETE (5-hydroxyeicosatetraenoic acid). The absorption at 234 nm was measured with a UV absorption monitor and the amount of 5-HETE produced was determined from the peak area corrected by the peak of the internal standard. The test was carried out using the test compound at various concentrations and the concentration that inhibits 50% enzyme activity was determined as the inhibitory activity.

b) Method for determining the inhibitory activity against platelet 12-lipoxygenase:

Lipoxygenase activity was determined by a modification of the method of D.H. Nugtern et al. [Biochim. Biophys. Acta, 380, 299 (1975)]. That is, a preparation prepared from bovine platelets according to the method described in the above reference was used as the source of 12-lipoxygenase. The enzyme preparation was contacted with a test compound in 60 µl of 0.17 M Tris-hydrochloride buffer (pH 7.4) at 30° C for 5 minutes. Then, 50 µl of 60 µM arachidonic acid was added to the reaction mixture and the mixture was allowed to stand at 30° C for 10 minutes. To the mixture were added 200 µl of methanol and 13-hydroxylinoleic acid as the internal standard, followed by thorough shaking. The mixture was allowed to stand at -20° C for 30 minutes and then centrifuged at 12000 r.p.m. for 10 minutes. After the supernatant was evaporated to dryness in a stream of nitrogen, 200 µl of 75% aqueous methanol was added to the residue. The resulting solution (100 µl) was subjected to high performance liquid chromatography to quantitatively determine 12-HETE (12-hydroxyeicosatetraenoic acid). The absorption at 234 nm was measured with a UV absorption monitor and the amount of 12-HETE produced was determined from the peak area corrected by the peak of the internal standard. The test was carried out using the test compound at various concentrations and the concentration that inhibits 50% enzyme activity was determined as the inhibitory activity.

The results are shown in Table 2.

Table 2

| Inhibitory Activity $IC_{50}$ (µM) | | |
|---|---|---|
| Compound No. | 5-Lipoxygenase | 12-Lipoxygenase |
| 1 | 0.02 | |
| 2 | 0.02 | |
| 3 | 0.02 | |
| 4 | 0.04 | |
| 5 | 0.036 | 0.06 |
| 6 | 0.025 | 0.01 |
| 7 | 0.038 | 0.01 |
| 10 | 0.034 | 0.014 |
| 11 | 0.044 | 0.003 |
| 12 | 0.3 | 0.036 |

**Claims**

1. Hydroxamic acid derivatives of the formula I
$R^1\text{-}X\text{-}(CH_2)_\ell\text{-}R^2$
wherein either:
$R^2$ is

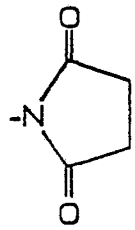 , 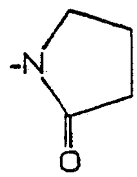 , 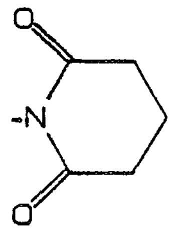 , or 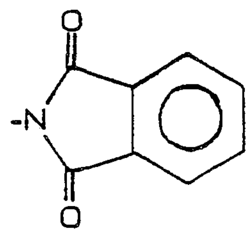

and $R^1$ is $C_1$-$C_{15}$ alkyl,

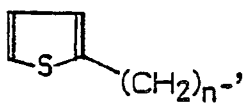 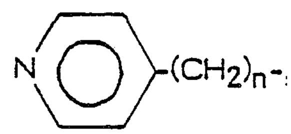

phenyl, or $R^3\text{-}\underset{\underset{O}{\|}}{C}\text{-}(CH_2)_m\text{-}$,

where $R^3$ is hydroxy, $C_1$-$C_5$ alkoxy or phenylamino;
or $R^2$ is H and $R^1$ is

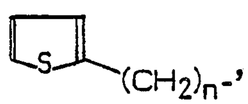 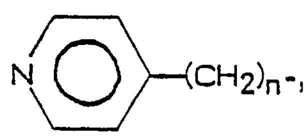

or $R^3\text{-}\underset{\underset{O}{\|}}{C}\text{-}(CH_2)_m\text{-}$,

where $R^3$ is as defined above;
X is $\text{-}CO\underset{\underset{OH}{|}}{N}$ or $\text{-}\underset{\underset{OH}{|}}{N}CO\text{-}$;
$\ell$ is an integer of 1 to 15;
n is 0 or an integer of 1 to 4;
m is an integer of 1 to 4;
with the proviso that, in either case, when $R^1$ is $R^3\text{-}\underset{\underset{O}{\|}}{C}\text{-}(CH_2)_m\text{-}$,

then X is $\text{-}CO\underset{\underset{OH}{|}}{N}$ ;

and their acid and base addition salts.

2. Hydroxamic acid derivatives of the formula IA:
$R^1\text{-}CO\underset{\underset{OH}{|}}{N}\text{-}(CH_2)_{11}H$

where $R^1$ is methyl, n-propyl, or phenyl;
and their acid and base addition salts.

3. Compounds Nos. 1 to 6 as identified herein.

4. Compound Nos. 7 to 14 as identified herein.

5. A pharmaceutical preparation comprising a compound of formula I, as defined in claim 1, or a pharmaceutically acceptable acid or base addition salt, in admixture with a pharmaceutically acceptable carrier or diluent.

6. A pharmaceutical preparation according to claim 5, wherein said compound is any one of compounds Nos. 1 to 6 as identified herein.

7. For use in the manufacture of pharmaceutical preparations having lipoxygenase inhibitory activity,

compounds of the formula IA defined in claim 2, and their pharmacuetically acceptable acid and base addition salts.

8. For use in the manufacture of pharmaceutical preparations for the treatment of bronchial asthma, allergic rhinitis, urticaria and other allergies, ischemic heart disease, hypertension, ischemic brain disease, arteriosclerosis or inflammatory disease, compounds of formula IA, as defined in claim 2, and their pharmaceutically acceptable acid and base addition salts.

9. For the uses specified in claim 7 or 8, the compounds Nos. 7 to 14 identified herein, and their pharmaceutically acceptable acid and base addition salts.

European Patent Office

# EUROPEAN SEARCH REPORT

Application Number

EP 89 31 0428

## DOCUMENTS CONSIDERED TO BE RELEVANT

| Category | Citation of document with indication, where appropriate, of relevant passages | Relevant to claim | CLASSIFICATION OF THE APPLICATION (Int. Cl.5) |
|---|---|---|---|
| A,D | US-A-4 604 407 (M.F. HASLANGER et al.) * claims 1,10; abstract * | 1,5,7,8 | C 07 D 207/404<br>C 07 D 207/38<br>C 07 D 209/48<br>C 07 D 211/88<br>C 07 D 401/12<br>C 07 D 409/12<br>C 07 D 333/24<br>C 07 D 333/38<br>A 61 K 31/40<br>A 61 K 31/44<br>A 61 K 31/38 |
| A | EP-A-0 199 151 (ABBOTT LABORATORIES) * claims 1,14 *; & US - A - 4623661 (Cat. A,D) | 1,5,7 | |
| A | EP-A-0 199 152 (ABBOTT LABORATORIES) * claims 1,9 * | 1,5,7 | |
| A | EP-A-0 273 451 (BRISTOL-MYERS CO.) * claims 1,17 * | 1,5,7 | |
| A,D<br>P | EP-A-0 320 000 (KYOWA HAKKO KOGYO CO.) * claims 1,7; abstract * | 1,5,7 | |
| A | US-A-4 077 998 (D.C. FESSLER et al.) * column 1, lines 50-55, starting compound in formula scheme; column 2, lines 39-54 * | 1 | |

TECHNICAL FIELDS SEARCHED (Int. Cl.5)

C 07 C 259/00
C 07 D 207/00
C 07 D 209/00
C 07 D 211/00
C 07 D 213/00
C 07 D 333/00
C 07 D 401/00
C 07 D 409/00

The present search report has been drawn up for all claims

| Place of search | Date of completion of the search | Examiner |
|---|---|---|
| BERLIN | 09-01-1990 | HASS C V F |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P0401)